# EUROPEAN PATENT APPLICATION

(11) **EP 4 450 084 A1**
(43) Date of publication of application: **23.10.2024**
(21) Application number: 23382365.7
(22) Date of filing: 19.04.2023
(51) Int. Cl.: A61K 39/12, A61K 39/008, A61P 31/14, A61P 33/02, A61K 39/00

(54) **THE LARGE EXTRACELLULAR LOOP (LEL) OF CD9 AS AN IMMUNOGENIC ADJUVANT IN LIPID NANOVESICLES**

(71) Applicant: Universidad Autónoma de Madrid, 28049 Madrid (ES)
(72) Inventor: YÁÑEZ MÓ, María, 28049 Madrid (ES); SOTO ÁLVAREZ, Manuel, 28049 Madrid (ES)
(74) Representative: Hoffmann Eitle

(57) **Abstract**

The present invention refers to a pharmaceutical composition (from hereinafter pharmaceutical composition of the invention), preferably a vaccine composition, comprising a lipid-based nanoparticle and optionally an excipient, wherein the lipid-based nanoparticle comprises the large extracellular loop (LEL) of CD9 of SEQ ID NO 2 displayed on its surface or an amino acid sequence having at least 75%, 80%, 85%, 90%, 91%, 92%, 93%, 94%, 95%, 96%, 97%, 98%, 99% or 100% identity to SEQ ID NO 2 displayed on its surface. That is, the large extracellular loop (LEL) of CD9 of SEQ ID NO 2, or a sequence having the indicated sequence identity, is associated with or displayed on the exterior surface of the lipid nanoparticle.

## Description

### TECHNICAL FIELD

The present invention generally relates to the field of vaccine compositions and methods of making and using the same. In particular, the present invention is directed to an immunogenic composition forming a vaccine, and its medical use.

### BACKGROUND ART

A significant number of infectious agents have been described that can cause contagious diseases in humans. Vaccines are the best way to prevent and treat these infectious diseases. To date, numerous vaccines that protect against these infectious agents have been approved. Current vaccines induce an immune response based on the production of antibodies. In some cases, this protection loses effectiveness over time, requiring new doses of vaccination to continue to be effective. On the other hand, this type of humoral response is not protective against some infectious agents. In fact, there is currently no vaccine for use in humans that protects against diseases caused by unicellular or multicellular parasites, in particular, vector infectious diseases transmitted by the bite of arthropods and that are currently spreading significantly.

The present invention provides for a synthetic vaccination platform, adaptable to various pathogens and even tumours and with a defined composition. Being an exosome mimetic, uptake by antigen-presenting phagocytic cells is enhanced, thus causing a potent and long-lasting response. Our proof of concept has demonstrated the enhancing effect of exosome mimetics in two infectious disease models: Leishmania and SARS-CoV-2 at the level of IgG production.

### BRIEF DESCRIPTION OF THE FIGURES

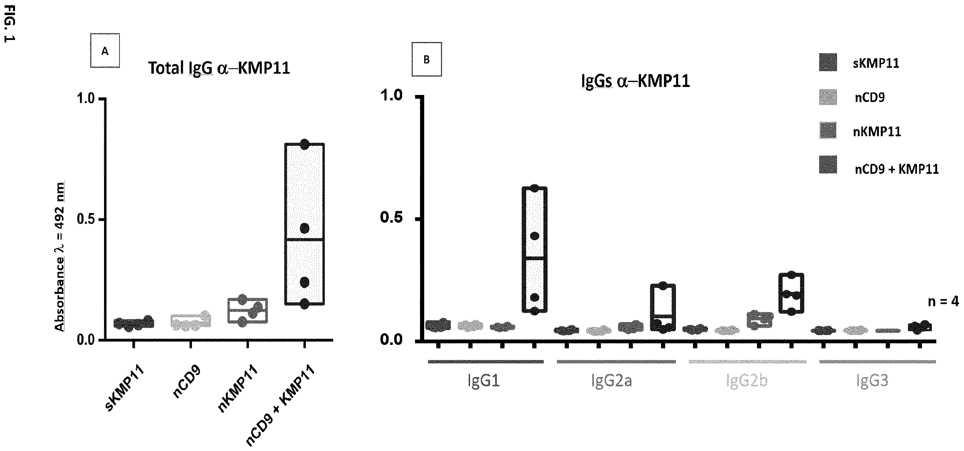

**Figure 1****: Antibody response against a *Leishmania* antigen produced as a recombinant protein: Kinetoplastid membrane protein of 11 kDa (KMP-11).** A. The quantification of anti-KMP-11 total IgG present in the serum of mice immunized with sKMP-11, nCD9, nKMP-11, nCD9 + KMP-11 is depicted. B. Quantification of the different anti-KMP-11 IgG Isotypes present in the serum of mice immunized with sKMP-11, nCD9, nKMP-11, nCD9 + KMP-11. The sera of immunized mice were drawn seven weeks after the first inoculation and analyzed by ELISA. Data corresponds to the signal obtained with a serum dilution 1 :2000. s (soluble) n (nanovesicle).

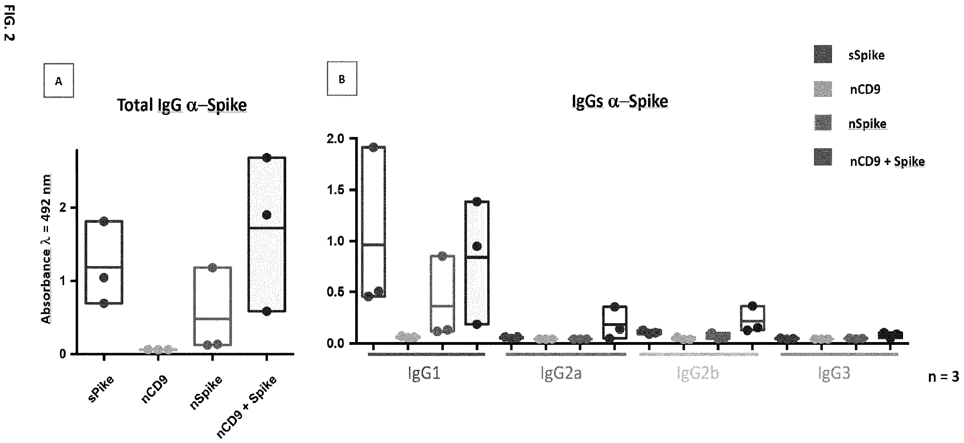

**Figure 2****: Antibody response against the Spike protein of the SARS-Cov2 produced as a recombinant protein** A. The quantification of anti-Spike total IgG present in the serum of mice immunized with sSpike, nCD9, nSpike, nCD9 + Spike is depicted. B. Quantification of the different anti-Spike IgG Isotypes present in the serum of mice immunized with sSpike, nCD9, nSpike, nCD9 + Spike. The sera of immunized mice were drawn seven weeks after the first inoculation and analysed by ELISA. Data corresponds to the signal obtained with a serum dilution 1:2000. s (soluble) n (nanovesicle)

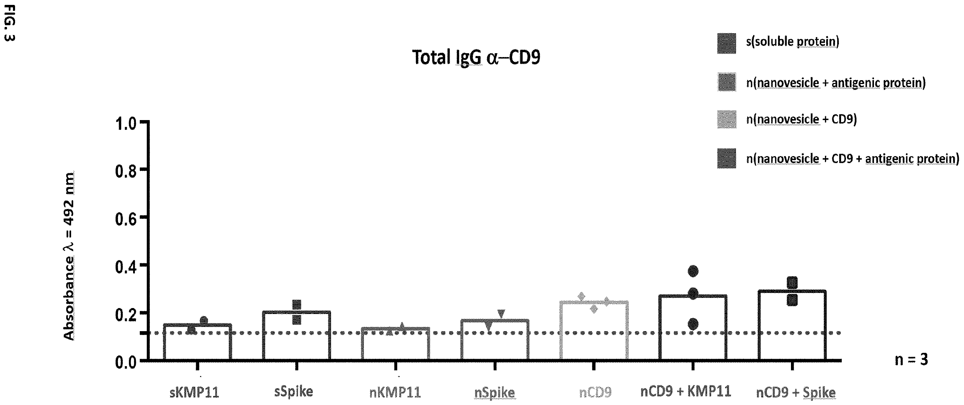

**Figure 3****.** Total anti-CD9 IgG detected in the serum of mice immunized with sKMP-11, sSpike, nKMP-11, nSpike, nCD9 + KMP-11 and nCD9 + Spike. Serum from immunized mice was drawn 7 weeks after the first injection and analyzed by ELISA. The red dotted indicates the mean value of the absorbance in the serum of pre-immune mice (n= 7). Serum dilution 1/2000.

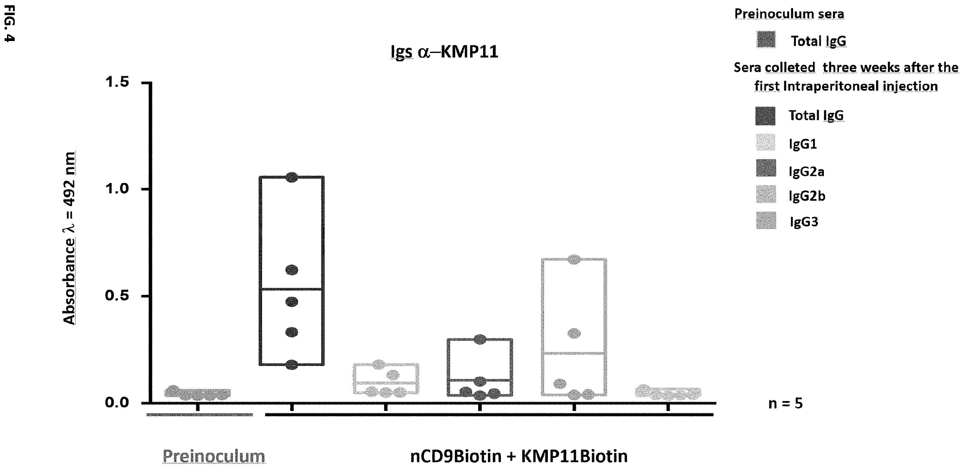

**Figure 4****:** Quantification of anti-KMP-11 Total IgG present in the serum of mice immunized with nanovesicles decorated with AviCD9Avi + biotinylated KMP-11. Preinoculum: serum extracted from blood of mice before first inoculation. Sera from immunized mice was drawn 3 weeks after inoculation and was analyzed by ELISA. Serum dilution 1/100.

### DEFINITIONS

Unless defined otherwise, all technical and scientific terms used herein have the same meaning as commonly understood by those of ordinary skill in the art to which this invention pertains. The following references provide one of skill with a general definition of many of the terms used in this invention: Academic Press Dictionary of Science and Technology , Morris (Ed.), Academic Press (Ist ed., 1992); Oxford Dictionary of Biochemistry and Molecular Biology, Smith et al. (Eds.), Oxford University Press (revised ed., 2000); Encyclopaedic Dictionary of Chemistry, Kumar (Ed.), Anmol Publications Pvt. Ltd. (2002); Dictionary of Microbiology and Molecular Biology, Singleton et al. (Eds.), John Wiley & Sons (3rd ed., 2002); Dictionary of Chemistry, Hunt (Ed.), Routledge (Ist ed., 1999); Dictionary of Pharmaceutical Medicine, Nahler (Ed.), Springer-Verlag Telos (1994); Dictionary of Organic Chemistry, Kumar and Anandand (Eds.), Anmol Publications Pvt. Ltd. (2002); and A Dictionary of Biology (Oxford Paperback Reference) , Martin and Hine (Eds.), Oxford University Press (4th ed., 2000).

Further clarifications of some of these terms as they apply specifically to this invention are provided herein.

As used herein, the singular forms "a," "an," and "the," refer to both the singular as well as plural, unless the context clearly indicates otherwise.

As used herein, the terms "antigen" or "immunogen" are used interchangeably to refer to a substance, typically a protein, which is capable of inducing an immune response in a subject. The term also refers to proteins that are immunologically active in the sense that once administered to a subject is able to evoke an immune response of the humoral and/or cellular type directed against that protein. Unless otherwise noted, the term "vaccine immunogen" is used interchangeably with "protein antigen" or "immunogen polypeptide".

As used herein, the term "effective amount" of a vaccine or other agent is the amount sufficient to generate a desired response, such as reduce or eliminate a sign or symptom of a condition or disease, such as *leishmaniasis* or COVID-19. For instance, this can be the amount necessary to inhibit parasite or viral replication or to measurably alter outward symptoms of the parasite or viral infection. In general, this amount will be sufficient to measurably inhibit virus replication or infectivity. When administered to a subject, a dosage will generally be used that will achieve target tissue concentrations that has been shown to achieve in vitro inhibition of parasite or viral replication. In some embodiments, an "effective amount" is one that treats (including prophylaxis) one or more symptoms and/or underlying causes of any of a disorder or disease. In some embodiments, an effective amount is a therapeutically effective amount. In some embodiments, an effective amount is an amount that prevents one or more signs or symptoms of a particular disease or condition from developing, such as one or more signs or symptoms associated with *leishmaniasis* or COVID-19.

Immunogen is a protein or a portion thereof that is capable of inducing an immune response in a mammal, such as a mammal infected or at risk of infection with a pathogen. Administration of an immunogen can lead to protective immunity and/or proactive immunity against a pathogen of interest.

Immune response refers to a response of a cell of the immune system, such as a B cell, T cell, or monocyte, to a stimulus. In some embodiment, the response is specific for a particular antigen (an "antigen-specific response"). In some embodiments, an immune response is a T cell response, such as a CD4⁺ response or a CD8⁺ response. In some other embodiments, the response is a B cell response, and results in the production of specific antibodies.

Immunogenic composition refers to a composition comprising an immunogenic polypeptide that induces a measurable CTL response against pathogens (virus, bacteria or parasites) expressing the immunogenic polypeptide, or induces a measurable B cell response (such as production of antibodies) against the immunogenic polypeptide.

Sequence identity or similarity between two or more nucleic acid sequences, or two or more amino acid sequences, is expressed in terms of the identity or similarity between the sequences. Sequence identity can be measured in terms of percentage identity; the higher the percentage, the more identical the sequences are.

Two sequences are "substantially identical" if two sequences have a specified percentage of amino acid residues or nucleotides that are the same (i.e., 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

The term "subject" refers to any animal classified as a mammal, e.g., human and non-human mammals. Examples of non-human animals include dogs, cats, cattle, horses, sheep, pigs, goats, rabbits, and etc. Unless otherwise noted, the terms "patient" or "subject" are used herein interchangeably. Preferably, the subject is human.

The term "treating" or "alleviating" includes the administration of compounds or agents to a subject to prevent or delay the onset of the symptoms, complications, or biochemical indicia of a disease, alleviating the symptoms or arresting or inhibiting further development of the disease, condition, or disorder. Subjects in need of treatment include those already suffering from the disease or disorder as well as those being at risk of developing the disorder. Treatment may be prophylactic (to prevent or delay the onset of the disease, or to prevent the manifestation of clinical or subclinical symptoms thereof) or related to the therapeutic suppression or alleviation of symptoms after the manifestation of the disease.

Vaccine refers to a pharmaceutical composition that elicits a prophylactic or therapeutic immune response in a subject. In some cases, the immune response is a protective immune response. Typically, a vaccine elicits an antigen-specific immune response to an antigen of a pathogen, for example a viral pathogen, or to a cellular constituent correlated with a pathological condition. A vaccine may include a polynucleotide (such as a nucleic acid encoding a disclosed antigen), a peptide or polypeptide (such as a disclosed antigen), a virus, a cell or one or more cellular constituents. In some embodiments of the invention, vaccines or vaccine immunogens or vaccine compositions are expressed from fusion constructs and self-assemble into nanoparticles displaying an immunogen polypeptide or protein on the surface.

### DESCRIPTION OF EMBODIMENTS

The present invention provides a vaccine platform based on vesicles, preferably nano or microvesicles (lipid-based nano or microparticles), whose surface is tailored with recombinant proteins. Recombinant proteins used can be classify as 1) **platform proteins,** which are part of the vaccine platform nano or microvesicles, and 2) **immunogenic proteins,** which come from any pathogen we want to induce protection against. These proteins or fragments thereof can be isolated from the pathogen, produced as heterologous proteins in other organism (bacteria, baculovirures, yeast, etc.) genetically modified to express genes encoding these proteins or chemically synthetized.

The **platform protein** tested for the present invention, see examples, comprises the large extracellular loop (LEL) of the tetraspanin CD9. The tetraspanin CD9 is among the most abundant membrane proteins on natural extracellular vesicles (EVs) and is routinely used as marker for their characterization. Within the tetraspanin protein structure, the large extracellular loop (LEL) is the most antigenic region and is where the majority of available anti-tetraspanin antibodies recognize.

Amino acid sequences of the LEL of the tetraspanin CD9 especially useful to practice the present invention can be selected from:
Mouse CD9 LEL (SEQ ID NO 1) 
THKDEVIKELQEFYKDTYQKLRSKDEPQRETLKAIHMALDCCGIAGPLEQFISDTCPKKQLLESFQVKPCPEAIS
EVFNNK
Human CD9 LEL (SEQ ID NO 2) 
SHKDEVIKEVQEFYKDTYNKLKTKDEPQRETLKAIHYALNCCGLAGGVEQFISDICPKKDVLETFTVKSCPDAIK
EVFDNK
Dog CD9 LEL (SEQ ID NO 3)

HKDEVIKEVQEFYKDTYSKLKSKDEPQRDTLKAIHYALNCCGLVGGVEQFISDICPQKDVLSSITVKPCPEAIKE
VFQNK

It is noted that the mouse and dog sequences share a sequence identity greater than 75% with the human sequence.

The **immunogenic** proteins tested for the present invention, see examples, where the sKMP-11 (from *Leishmania*) and sSpike proteins (from SARs-Cov-2 virus).

Recombinant KMP-11 from *Leishmania* parasite. Kinetoplastid membrane protein-11 (KMP-11) is a protein located on the cell membrane of kinetoplastid parasites. KMP-11 has a wide distribution and conservation among kinetoplastids what suggests an important role in the biology of the parasite. KMP-11 does not activate the immune response by itself when is injected in a soluble form (low immunogenicity).

Recombinant Spike, in the case of SARs-Cov-2 virus. Spike is the largest of the four major structural proteins found in coronaviruses. The spike protein assembles intro primers that form large structures, called spikes or peplomers, that project from the surface of the virion. Spike protein already has high immunogenicity in its soluble form.

In order to test the vaccine platform based on vesicles of the present invention, two different nanovesicles were assessed, depending on the tag of the recombinant proteins used, see examples. Surface modification of nanovesicles was performed by bioconjugation with streptavidin (SA) or avidin through thiol activated groups within the molecules of the bilayer, thereby generating a covalent bond between (strept)avidin molecules and the nanovesicles; or by the use of Niquel-carrying lipids and poly-His tagged proteins.

Decoration of the said surface modified lipid nanovesicles with recombinant proteins was carried out with tagged recombinant KMP-11, CD9mLEL, or Spike as well as different combinations thereof, as described in the examples of the present invention.

Once the lipid nanovesicles were decorated with the recombinant proteins, solutions of soluble recombinant immunogenic proteins (sKMP-11 and sSpike), or nanovesicles decorated only with recombinant CD9LEL protein (nCD9), nanovesicles decorated only with antigenic protein (nKMP-11 or nSpike) and nanovesicles decorated with both antigenic protein and CD9 protein (nCD9+ KMP-11 and nCD9 + Spike) were prepared in PBS. 400 µl at different preparations containing in all cases 10 µg of antigenic protein, and were intraperitoneally injected in mice using a 22-G needle.

ELISAs were established for detection of serum total IgG and IgG isotypes (IgG1, IgG2a, IgG2b and IgG3) specific for KMP-11, Spike and CD9.

As shown in figure 1A, quantification of anti-KMP-11 total IgG present in the serum of mice immunized with sKMP-11, nCD9, nKMP-11, nCD9 + KMP-11 is depicted. In figure 1B, quantification of the different anti-KMP-11 IgG Isotypes present in the serum of mice immunized with sKMP-11, nCD9, nKMP-11, nCD9 + KMP-11 is depicted. Interestingly, the total IgG is significantly increased primarily when CD9 LEL is used in combination with the specific vaccine antigen (KMP-11). That is, CD9 LEL acts as an immunologic adjuvant.

As used in the present invention, the adjuvant is a substance, in the present invention CD9 LEL, that increases or modulates the immune response to a vaccine. More specifically, an immunologic adjuvant, as used in the present invention, is defined as any substance, in the present invention CD9 LEL, that acts to accelerate, prolong, or enhance antigen-specific immune responses when used in combination with specific vaccine antigens.

As shown in figure 2, quantification of anti-Spike total IgG present in the serum of mice immunized with sSpike, nCD9, nSpike, nCD9 + Spike is depicted. In figure 2B, quantification of the different anti-Spike IgG Isotypes present in the serum of mice immunized with sSpike, nCD9, nSpike, nCD9 + Spike is depicted. Interestingly, the total IgG is significantly increased, even above soluble Spike, when CD9 LEL is used in combination with the specific vaccine antigen (Spike). That is, again, CD9 LEL is an immunologic adjuvant,
Figures 3 demonstrates that there is no immunological response against the adjuvant molecule (CD9-LEL) which corresponds to the host CD9 sequence.

Figure 4 demonstrates that the adjuvant effect of CD9 LEL is observed independently of the tagging strategy employed (Biotin-tag to bind to avidin-carrying nanovesicles or His-tag to Ni₂⁺ carrying nanovesicles).

Therefore, a first aspect of the present invention refers to a pharmaceutical composition (from hereinafter pharmaceutical composition of the invention), preferably a vaccine composition, comprising a lipid-based micro or nanoparticle, preferably a nanovesicle, and optionally an excipient, wherein the lipid-based nanoparticle, preferably nanovesicle, comprises the large extracellular loop (LEL) of CD9 of SEQ ID NO 2 displayed on its surface or an amino acid sequence having at least 75%, 80%, 85%, 90%, 91%, 92%, 93%, 94%, 95%, 96%, 97%, 98%, 99% or 100% identity to SEQ ID NO 2 displayed on its surface. That is, the large extracellular loop (LEL) of CD9 of SEQ ID NO 2, or a sequence having the indicated sequence identity, is associated with or displayed on the exterior surface of the lipid nanoparticle, preferably nanovesicle.

In an embodiment, the pharmaceutical composition, preferably a vaccine composition, of the present invention preferably comprises associated to or displayed on the surface of the lipid-based nanoparticle, preferably nanovesicle, **platform proteins** and preferably **polypeptide immunogens,** wherein the polypeptide immunogens come from any microorganism we want to induce protection against, and the **platform protein** is the large extracellular loop (LEL) of the host CD9 of SEQ ID NO 2 or an amino acid sequence at least 75%, 80%, 85%, 90%, 91%, 92%, 93%, 94%, 95%, 96%, 97%, 98%, 99% or 100% identical to SEQ ID NO 2. For the purpose of the present invention, the polypeptide immunogens shall also be referred to as protein, polypeptide or peptide immunogens, preferably as polypeptide immunogens. It is noted that, in the present invention, the large extracellular loop (LEL) of CD9 of SEQ ID NO 2 or the amino acid sequence at least 75%, 80%, 85%, 90%, 91%, 92%, 93%, 94%, 95%, 96%, 97%, 98%, 99% or 100% identical to SEQ ID NO 2, associated to or displayed on its surface of the lipid-based nanoparticle, preferably nanovesicle, is used as an immunologic adjuvant of the polypeptide immunogens also displayed on the surface of the lipid nanoparticle, preferably nanovesicle. It is thus preferably noted that the **polypeptide immunogens according to the present invention are** different from the **platform proteins, namely different from the** large extracellular loop (LEL) of CD9 of SEQ ID NO 2 and different from an amino acid sequence having at least 75%, 80%, 85%, 90%, 91%, 92%, 93%, 94%, 95%, 96%, 97%, 98%, 99% or 100% identity to SEQ ID NO 2.

Any polypeptide immunogens can be used in the pharmaceutical composition of the invention, as long as it is preferably different from the platform proteins as defined in the present invention. These include any proteins or polypeptides from pathogens against which an elicited immune response may be desired. Thus, the vaccine compositions of the invention can utilize immunogen polypeptides that are derived from any viruses, bacteria or other pathogenic organisms such as parasites. Suitable immunogen polypeptides for the invention can also be derived from non-pathogenic species, including human proteins, against which an elicited immune response may have a therapeutic effect, alleviate disease symptoms, or improve general health (as for example in the case of tumoral antigens). In general, the immunogen polypeptide can be any structural or functional polypeptide or peptide that contains at least about 10 amino acid residues. In some embodiments, the immunogen polypeptides contains between about 10 to about 10,000 amino acid residues in length. In some embodiments, the immunogen polypeptides contains between about 25 to about 2,000 amino acid residues in length. In some embodiments, the immunogen polypeptides contains about 50 to about 500 amino acid residues in length. Thus, the immunogen polypeptides or proteins suitable for the invention can have a molecular weight of from about 1 kDa to about 1,000 kDa, and preferably from about 2.5 kDa to about 250 kDa. In some more preferred embodiments, the employed immunogen polypeptide has a molecular weight of about 5 kDa to about 25 kDa or 50 kDa.

In some embodiments, the immunogen polypeptide or protein used in the vaccine compositions of the invention can be derived from a viral surface or core protein (target polypeptide). There are many known viral proteins that are important for viral infection of host cells. Examples include, but are not limited to, glycoproteins (or surface antigens, e.g., GP120 and GP41) and capsid proteins (or structural proteins, e.g., P24 protein) of HIV; surface antigens or core proteins of hepatitis A, B, C, D or E virus (e.g., small hepatitis B virus surface antigen (S-HBsAg) and the core proteins of hepatitis C virus, NS3, NS4 and NS5 antigens); glycoproteins gp350/220 of Epstein- Barr virus (EBV), glycoprotein (G-protein) or the fusion protein (F-protein) of respiratory syncytial virus (RSV); surface and core proteins of herpes simplex virus HSV-1 and HSV-2 (e.g., glycoprotein D from HSV-2), surface proteins (e.g., gB, gC, gD, gH and gL) of poliovirus, envelope glycoproteins hemagglutinin (H) and fusion protein (F) of measles virus (MV), glycoprotein G of lymphocytic choriomeningitis virus (LCMV), fiber and penton base proteins of adenoviruses, S spikes of coronaviruses, envelope (E) proteins of flaviviruses such as Dengue virus, yellow fever virus, and Zika virus, and non-enveloped capsid proteins of picomaviruses. Preferably, the immunogen polypeptides are selected from the recombinant Spike, in the case of SARs-Cov-2 virus.

In some embodiments, the immunogen polypeptide or protein used in the vaccine compositions of the invention can be derived from a non-viral target. These include immunogens that can be obtained from any non-viral pathogens (e.g., bacterial pathogens) as well as parasitic organisms inside mammalian hosts such as human. In some embodiments, bacterial proteins that are important for bacterial infections are suitable for obtaining immunogen polypeptides in the vaccine design of the invention. Suitable immunogens can be any proteins and polypeptides that are derived from structural proteins of the bacteria. In some embodiments, parasitic proteins that are important for parasite transmission, reproduction in the hosts, and life cycle are suitable for obtaining immunogen polypeptides in the vaccine design of the invention. Suitable immunogens can be any proteins and polypeptides that are derived from structural proteins of the parasites. Preferably, the immunogen polypeptides are selected from recombinant KMP-11 from *Leishmania* parasite.

In some embodiments, the employed immunogen polypeptide can be an endogenous protein from a mammalian host (e.g., human), against which an elicited immune response is desired. These include, e.g., PCSK9 for regulating cholesterol level, and ghrelin for controlling appetite. Various other mammalian proteins can also be used to obtain suitable immunogen polypeptides for constructing vaccines in accordance with the design of the invention. In some embodiments, the non-viral target for vaccine design can be other proteins implicated in human diseases. These include proteins that are involved in the development of cancers. Examples of cancer related immunogens also include non-mutated self antigens, e.g., MAGE-A3, Melan-A/Martl, gplOO, Her2/Neu, and NY-ESO-1. In some additional embodiments, the immunogen polypeptides or proteins for use in vaccine compositions of the invention include proteins implicated in other chronic human diseases or disorders. Examples of such human target include, e.g., Ang-II for hypertension, TNF-a for inflammations, IL-9 for pathogen-induced eosinophilia, IL-5 for asthma, N-methyl-D-aspartate receptor- 1 for stroke and human chorionic gonadotropin (hCG) for decreasing hormone levels.

As indicated above, the pharmaceutical composition of the present invention, preferably the vaccine composition, comprises a lipid-based nanoparticle, preferably nanovesicle. A lipid-based nanoparticle is a liposome, an exosome, lipid preparations, or another lipid-based nanoparticle, such as a lipid-based vesicles or nanovesicles (e.g., a DOTAP:cholesterol vesicles or nanovesicles).

Lipid-based nanoparticles, preferably nanovesicles, may be positively charged, negatively charged or neutral.

A "liposome" is a generic term encompassing a variety of single and multilamellar lipid vehicles formed by the generation of enclosed lipid bilayers or aggregates. Liposomes may be characterized as having vesicular structures with a bilayer membrane, generally comprising a phospholipid or a combination of phospholipids, and an inner medium that generally comprises an aqueous composition.

Liposomes provided herein include unilamellar liposomes, multilamellar liposomes, and multivesicular liposomes. Liposomes provided herein may be positively charged, negatively charged, or neutrally charged. In certain embodiments, the liposomes are neutral in charge. A multilamellar liposome has multiple lipid layers separated by aqueous medium. Such liposomes form spontaneously when lipids comprising phospholipids are suspended in an excess of aqueous solution. The lipid components undergo self-rearrangement before the formation of closed structures and entrap water and dissolved solutes between the lipid bilayers. Lipophilic molecules or molecules with lipophilic regions may also dissolve in or associate with the lipid bilayer.

A liposome used according to the present embodiments can be made by different methods, as would be known to one of ordinary skill in the art.

The terms "vesicle", "nanovesicle" and "exosomes," as used herein, refer to a membranous particle having a diameter (or largest dimension where the particles is not spheroid) of between about 10 nm to about 5000 nm, more typically between 30 nm and 1000 nm, and most typically between about 50 nm and 750 nm, wherein at least part of the membrane of the exosomes could optionally be directly obtained from a cell. Most commonly, exosomes will have a size (average diameter) that is up to 5% of the size of the donor cell. Preferably, the nanovesicle has a diameter (or largest dimension where the particles is not spheroid) of between about 10 nm to about 150 nm.

The term "exosome mimetic" as used herein refers to a liposome that carries on its surface some abundant exosome surface molecules or peptides from these molecules, such as the CD9LEL, and can artificially be made by decorating the surface of a liposome or nanovesicle with these exosome molecules.

As indicated above, the pharmaceutical composition of the present invention, preferably the vaccine composition, comprises a lipid-based nanoparticle, preferably nanovesicle, that comprises associated to or displayed on its surface **platform proteins** and preferably polypeptide immunogens, wherein the polypeptide immunogens come from any microorganism we want to induce protection against, and the **platform protein** is the large extracellular loop (LEL) of CD9 of SEQ ID NO 2 or an amino acid sequence at least 75%, 80%, 85%, 90%, 91%, 92%, 93%, 94%, 95%, 96%, 97%, 98%, 99% or 100% identical to SEQ ID NO 2. It is noted that such association or displayed on the lipid nanoparticles, preferably nanovesicles, can be performed by any method known to the person skilled in the art, preferably by bioconjugation with a molecule such as streptavidin (SA) or avidin through activated groups, such as thiol groups, within the molecules of the bilayer, thereby generating a covalent bond between molecules, such as (strept)avidin molecules, and the lipid nanoparticles, preferably nanovesicles. Preferably, such association between the **platform proteins** and preferably also the polypeptide immunogens and the lipid nanoparticles, preferably nanovesicles, is preferably based on linking or binding such **platform proteins** and preferably polypeptide immunogens via a biotin tag to the strept(avidin) molecules coupled to the lipid components of the lipid-based nanoparticles, preferably nanovesicles.

Preferably by the insertion of NTA Ni2+ carrying lipids in the composition of the nanoparticle or nanovesicle, and preferably, such association between the **platform proteins** and preferably also the polypeptide immunogens and the lipid nanoparticle or nanovesicle, is preferably based on His tags coordination with the Ni2+ carried on the lipid component of the lipid-based nanoparticle or nanovesicle.

Preferably, the lipid component is one or more of phosphatidylcholine, cholesterol, phosphatidylglycerol, phosphatidyl- ethanolamine, phosphatidylserine, phosphatidic acid, sphingomyelin or derivatives of these lipids. More preferably, the lipid components are phosphatidylcholine and cholesterol.

In a preferred embodiment, the pharmaceutical composition of the present invention is a vaccine composition optionally comprising a physiologically-acceptable excipient.

A second aspect of the invention refers to the pharmaceutical composition, preferably a vaccine composition, of the present invention comprising associated to or displayed on the surface of the lipid-based nanoparticles, preferably nanovesicles, **platform proteins** and **polypeptide immunogens,** wherein the polypeptide immunogens come from any microorganism we want to induce protection against, and the **platform protein** is the large extracellular loop (LEL) of CD9 of SEQ ID NO 2 or an amino acid sequence at least 75%, 80%, 85%, 90%, 91%, 92%, 93%, 94%, 95%, 96%, 97%, 98%, 99% or 100% identical to SEQ ID NO 2, for use as a medicament.

In a preferred embodiment of the second aspect of the invention, the pharmaceutical composition, preferably a vaccine composition, of the present invention is for use as an immunogenic composition capable of inducing a measurable CTL response against the immunogenic polypeptide displayed on the surface of the lipid nanoparticles, preferably nanovesicles, and/or induces a measurable B cell response (such as production of antibodies) against the immunogenic polypeptide.

In another preferred embodiment of the second aspect of the invention, the large extracellular loop (LEL) of CD9 of SEQ ID NO 2 or the amino acid sequence at least 75%, 80%, 85%, 90%, 91%, 92%, 93%, 94%, 95%, 96%, 97%, 98%, 99% or 100% identical to SEQ ID NO 2, associated to or displayed on the surface of the lipid-based nanoparticles, preferably nanovesicles, is for use as an immunologic adjuvant of the polypeptide immunogens also displayed on the surface of the lipid nanoparticles, preferably nanovesicles.

In yet another preferred embodiment of the second aspect of the invention, the pharmaceutical composition, preferably a vaccine composition, of the present invention is for use as an immunogenic composition capable of inducing a measurable CTL response against the immunogenic polypeptide displayed on the surface of the lipid nanoparticle, preferably nanovesicle, and/or induces a measurable B cell response (such as production of antibodies) against the immunogenic polypeptide, and the large extracellular loop (LEL) of CD9 of SEQ ID NO 2 or the amino acid sequence at least 75%, 80%, 85%, 90%, 91%, 92%, 93%, 94%, 95%, 96%, 97%, 98%, 99% or 100% identical to SEQ ID NO 2, associated to or displayed on its surface of the lipid-based nanoparticle, preferably nanovesicle, is for use as an immunologic adjuvant of the polypeptide immunogens displayed on the surface of the lipid nanoparticle, preferably nanovesicle.

It is herein noted that, for the second aspect of the present invention as well as for any or its preferred embodiments, any polypeptide immunogens can be used in the pharmaceutical composition of the invention. These include any proteins or polypeptides from pathogens against which an elicited immune response may be desired. Thus, the vaccine compositions of the invention can utilize immunogen polypeptides that are derived from any viruses, bacteria or other pathogenic organisms such as unicellular or multicellular parasites, in particular, vector infectious diseases transmitted by the bite of arthropods and that are currently spreading significantly. Suitable immunogen polypeptides for the invention can also be derived from non-pathogenic species, including human proteins, against which an elicited immune response may have a therapeutic effect, alleviate disease symptoms, or improve general health. In general, the immunogen polypeptide can be any structural or functional polypeptide or peptide that contains at least about 10 amino acid residues. In some embodiments, the immunogen polypeptides contains between about 10 to about 10,000 amino acid residues in length. In some embodiments, the immunogen polypeptides contains between about 25 to about 2,000 amino acid residues in length. In some embodiments, the immunogen polypeptides contains about 50 to about 500 amino acid residues in length. Thus, the immunogen polypeptides or proteins suitable for the invention can have a molecular weight of from about 1 kDa to about 1,000 kDa, and preferably from about 2.5 kDa to about 250 kDa. In some more preferred embodiments, the employed immunogen polypeptide has a molecular weight of about 5 kDa to about 25 kDa or 50 kDa.

In some embodiments, the immunogen polypeptide or protein used in the vaccine compositions of the invention can be derived from a viral surface or core protein (target polypeptide). There are many known viral proteins that are important for viral infection of host cells. Examples include, but are not limited to, glycoproteins (or surface antigens, e.g., GP120 and GP41) and capsid proteins (or structural proteins, e.g., P24 protein) of HIV; surface antigens or core proteins of hepatitis A, B, C, D or E virus (e.g., small hepatitis B virus surface antigen (S-HBsAg) and the core proteins of hepatitis C virus, NS3, NS4 and NS5 antigens); glycoproteins gp350/220 of Epstein- Barr virus (EBV), glycoprotein (G-protein) or the fusion protein (F-protein) of respiratory syncytial virus (RSV); surface and core proteins of herpes simplex virus HSV-1 and HSV-2 (e.g., glycoprotein D from HSV-2), surface proteins (e.g., gB, gC, gD, gH and gL) of poliovirus, envelope glycoproteins hemagglutinin (H) and fusion protein (F) of measles virus (MV), glycoprotein G of lymphocytic choriomeningitis virus (LCMV), fiber and penton base proteins of adenoviruses, S spikes of coronaviruses, envelope (E) proteins of flaviviruses such as Dengue virus, yellow fever virus, and Zika virus, and non-enveloped capsid proteins of picomaviruses. In some embodiments, viral immunogens suitable for the invention can be derived from viruses such as those utilizing the class-I fusion mechanism for infection. Preferably, the immunogen polypeptides are selected from recombinat Spike, in the case of SARs-Cov-2 virus.

In some embodiments, the immunogen polypeptide or protein used in the vaccine compositions of the invention can be derived from a non-viral target. These include immunogens that can be obtained from any non-viral pathogens (e.g., bacterial pathogens) as well as parasitic organisms inside mammalian hosts such as human. In some embodiments, bacterial proteins that are important for bacterial infections are suitable for obtaining immunogen polypeptides in the vaccine design of the invention. Suitable immunogens can be any proteins and polypeptides that are derived from structural proteins of the bacteria. In some embodiments, parasitic proteins that are important for parasite transmission, reproduction in the hosts, and life cycle are suitable for obtaining immunogen polypeptides in the vaccine design of the invention. Suitable immunogens can be any proteins and polypeptides that are derived from structural proteins of the parasites. Preferably, the immunogen polypeptides are selected from recombinant KMP-11 from *Leishmania* parasite.

In some embodiments, the employed immunogen polypeptide can be an endogenous protein from a mammalian host (e.g., human), against which an elicited immune response is desired. These include, e.g., PCSK9 for regulating cholesterol level, and ghrelin for controlling appetite. Various other mammalian proteins can also be used to obtain suitable immunogen polypeptides for constructing vaccines in accordance with the design of the invention. In some embodiments, the non-viral target for vaccine design can be other proteins implicated in human diseases. These include proteins that are involved in the development of cancers. Examples of cancer related immunogens also include non-mutated self antigens, e.g., MAGE-A3, Melan-A/Martl, gplOO, Her2/Neu, and NY-ESO-1. In some additional embodiments, the immunogen polypeptides or proteins for use in vaccine compositions of the invention include proteins implicated in other chronic human diseases or disorders. Examples of such human target include, e.g., Ang-II for hypertension, TNF-a for inflammations, IL-9 for pathogen-induced eosinophilia, IL-5 for asthma, N-methyl-D-aspartate receptor- 1 for stroke and human chorionic gonadotropin (hCG) for decreasing hormone levels.

The following examples merely illustrate the present invention but do not limit the same.

### EXAMPLES

### Construction of recombinant histidine tetraspanin LEL of mouse CD9 expression plasmid

HisCD9mLELHis sequence was cloned into the pGEX-4T2 expression vector. DNA sequence corresponding to the LEL of mouse CD9 tetraspanin was amplified by PCR with the primers listed below that included the His-Tag and restriction sites for XhoI and BamHI. Products of PCR amplification and the pGEX-4T2 vector were digested with XhoI and BamHI restriction enzymes, ligated and transformed into DH5α competent E. coli cells by heat shock. Ampicillin-resistant clones were expanded and verified by restriction pattern with XhoI and BamHI and DNA sequencing.

### HisCD9mLELHis Primers:

Forward
   GATGGATCCCATCATCACCATCACCATACCCACAAGGATGAGGTGATTAAAG
Reverse 
GATCCTCGAGTTAATGGTGATGGTGATGATGCTTGTTGTTGAAGACCTCACTGAT
G
Restriction site: BamHI; Restriction site: XhoI
His-Tag (6His)
Start of CD9 mouse LEL sequence (Forward); end of CD9 mouse LEL sequence (Reverse)
STOP Codon

### Construction of recombinant biotinylated tetraspanin LEL of mouse CD9 expression plasmid

AviCD9mLELAvi sequence was cloned into the pGEX-4T2 expression vector. DNA sequence corresponding to the LEL of CD9 mouse tetraspanin was amplified by PCR with the primers lister below that included the recognition sequences for BirA and restriction sites for XhoI and BamHI. Products of PCR amplification were inserted in the TOPO^{®} TA vector (Invitrogen) and transformed into supercompetent bacteria by heat shock. Ampicillin-resistant clones were expanded and sequenced. Validated clones were digested with XhoI and BamHI and ligated in the reading frame of GST in the final vector pGEX-4T2. Products of ligation were verified by restriction pattern with XhoI and BamHI.

### AviCD9mLELAvi Primers:

Forward 
GATGGATCCTCCGGCCTGAACGACATCTTCGAGGCTCAGAAAATCGAATGGCACG
AAACCCACAAGGATGAGGTGATTAAAG
Reverse 
GATCCTCGAGTTATTCGTGCCATTCGATTTTCTGAGCCTCGAAGATGTCGTTCAGA
CCGCCACCCTTGTTGTTGAAGACCTCACTGATG
Restriction site: BamHI; Restriction site: XhoI
AviTag (recognition site for the biotin ligase enzyme) BirA
Star CD9 mouse LEL sequence (Forward); end CD9 mouse LEL sequence (Reverse)
STOP Codon

### Expression and purification of recombinant tagged CD9mLEL

Protease-deficient super-competent Escherichia coli BL21 cells were transformed with either HisCD9mLELHis-pGEX-4T2 or AviCD9mLELAvi-pGEX-4T2 constructs. For Avi-tagged constructs bacteria were co-transformed with pBirAcm, an engineered pACYC184 plasmid with an IPTG inducible BirA gene to over-express the biotin ligase (isolated from the K12 E. coli strain AVB99, Avidity LLC). Colonies were selected and grown overnight in 10 mL of Luria-Bertani (LB) medium containing 0.1 mg/mL ampicillin (Normon) (and 20 µg/mL chloramphenicol (Sigma) for Avi-Tag). The seed culture was then transferred into 400 mL of fresh LB medium with antibiotics and cultured for 3 h at 37°C and 200 rpm. Isopropyl-beta-D-thiogalactopyranoside (IPTG, Sigma) was then added to a final concentration of 0.3 mM and bacterial culture continued for 2-3 h at 37°C and 200 rpm.

Cells were harvested by centrifugation at 6000 g for 15 min at 4°C. The bacterial pellet was resuspended in 20 mL of Tris-Buffered Saline (TBS) supplemented with protease inhibitors cocktail (Roche). Bacteria were disrupted using a homogenizer GEA Niro and bacterial lysates centrifuged at 18,000 g for 30 min at 4°C. Supernatant was collected and GST fusion proteins were purified by affinity chromatography using glutathione-sepharose 4B (GE Healthcare). Proteins were cleaved and eluted from GST using site specific protease thrombin (GE Healthcare). Benzamidine-sepharose (Sigma-Aldrich) was used for the removal of thrombin. Protein concentration of the preparations was measured with Micro BCA Protein Assay Kit (Pierce Company) following the manufacturer's instructions.

Samples along the purification process were boiled in Laemmli buffer and resolved by 12% SDS-PAGE. Gels were incubated with Coomassie Brilliant Blue for 15 min, followed by washes with destaining solution (20% methanol, 10% acetic acid) for 30 min.

### Immunogenic proteins

The immunogenic proteins tested were:
- Recombinant KMP-11 from *Leishmania* parasite. Kinetoplastid membrane protein-11 (KMP1) is one component of the cell membrane of kinetoplastid parasites. KMP-11 has a wide distribution and conservation among kinetoplastids what suggests an important role in the biology of the parasite. KMP-11 does not activate the immune response by itself when is injected in a soluble form (low immunogenicity).

KMP-11 gene encoding sequence was cloned into pQE30 vector (Qiagen). DNA sequence coding for the entire native protein was amplified by PCR from a genomic EMBL-3 phage containing the KMP-11 gene cluster, using primers listed below. BamHI and KpnI restriction sites to allow cloning and a stop codon in the antisense oligonucleotide was included. The PCR product was cloned into the pQE30 vector (QIAGEN).

The recombinant protein, HisKMP-11, possesses the whole 92 amino acid residues of the *Leishmania* KMP-11 protein plus 12 amino acids coded by the vector, including 6 histidines employed for affinity purification fused at the amino terminal region.

M15 E. coli cells were transformed with this plasmid for a high-level expression of the recombinant His-tagged protein. Bacteria were grown overnight in 10 mL of Luria-Bertani (LB) medium containing 0.1 mg/mL ampicillin (Normon) and 25 µg/ml kanamycin. The culture was then transferred into 500 mL of fresh LB medium with antibiotics and cultured for 2 h at 37°C and 200 rpm. IPTG (Sigma) was then added to a final concentration of 1 mM and bacterial culture continued for 4 h at 37°C and 200 rpm. Cells were harvested by centrifugation at 6000 g for 30 min at 4°C. The bacterial pellet was resuspended in 20 mL of Tris-Buffered Saline (TBS) supplemented with protease inhibitors cocktail (Roche). Bacteria were disrupted using a homogenizer GEA Niro. Bacterial lysates were centrifuged at 18,000 g for 30 min at 4°C. Supernatant was collected and HisKMP-11 was purified by a Ni-NTA affinity chromatography column (QIAGEN). The purity of the recombinant protein was analyzed in SDS/PAGE gels and posterior staining with Coomassie Brilliant Blue. Protein concentration was measured with Micro BCA Protein Assay Kit (Pierce Company) following the manufacturer's instructions.

For biotynilated recombinant KMP-11, HisKMP-11 at 1mg/ml was biotynilated in vitro with Sulfo-NHS-LC-Biotin (ThermoScientific) at 100 µg/ml in bicarbonate buffer solution, during 4 hours at room temperature and agitation. Biotynilated KMP-11 was dialyzed against PBS to remove unbound-biotin.

### HisKMP-11 Primers:

Forward
   CGGGATCCATGGCCACCACGTACGAGGAG
Reverse
   GGGGTACCTTACTTGGATGGGTACTGCGC
Restriction site: BamHI; Restriction site: KpnI
Star of KMP-11 sequence (Forward); end of KMP-11 sequence (Reverse)
STOP Codon
   - Recombinant Spike, in the case of SARs-Cov-2 virus. Spike is the largest of the four major structural proteins found in coronaviruses. The spike protein assembles intro primers that form large structures, called spikes or peplomers, that project from the surface of the virion. Spike protein already has high immunogenicity in its soluble form.

Recombinant Spike was donated by Dr Casasnovas. cDNA coding for soluble S (residues 1 to 1208) protein was cloned in the pcDNA3.1 vector for expression in HEK-293F cells using standard transfection methods. The construct contained the S signal sequence at the N-terminus, and a T4 fibritin trimerization sequence, a Flag epitope, and an 8xHis-tag at the C-terminus. The furin-recognition motif (RRAR) was replaced by the GSAS sequence and it contained the A942P, K986P, and V987P substitutions in the S2 portion. Proteins were purified by Ni-NTA affinity chromatography from transfected cell supernatants and they were transferred to 25 mM Hepes-buffer and 150 mM NaCl, pH 7.5, during concentration.

### Lipid nanovesicles

Two different nanovesicles have been assessed, depending on the tag of the recombinant proteins used.
**a)** For His-tagged recombinant proteins nanovesicles had the following formulation of lipids:
   - 50% 1,2-dioleoyl-sn-glycero-3-phosphocholine, DOPC (18:1 (Δ9-Cis) PC) (Avanti Polar Lipids)
   - 20% 1,2-dioleoyl-sn-glycero-3-phosphocholine, DOPG (18:1 (Δ9-Cis) PG) (Avanti Polar Lipids)
   - 28% cholesterol (Sigma)
   - 1%1,2-dioleoyl-sn-glycero-3-[(N-(5-amino-1-carboxypentyl)iminodiacetic acid)succinyl] (nickel salt) DGS-NTA(Ni) 18:1 (Avanti Polar Lipids)
   - 1% Atto 488 1,2-dipalmitoyl-sn-glycero-3- phosphoethanolamine DPPE (or 1% 1,2-dioleoyl-sn-glycero-3-phosphoethanolamine-N-(lissamine rhodamine B sulfonyl) (ammonium salt) 16:0 Liss Rhod PE) (Sigma).
   Lipids at the desired lipid ratio were mixed in chloroform solution and consecutively dried under a nitrogen stream until a lipid film is formed. This film was rehydrated in PBS for 15 min and vortexed during 5 min. The total lipid concentration of this nanovesicles was 3mM. The preformed multilamellar vesicles were extruded at least 11 times through nanopore channels (Mini-extruder, Avanti polar lipids; 100nm Whatman^{®} Nuclepore^{™} Track-Etched Membranes) to obtain unilamellar vesicles with a size range between 100-150 nm diameter. Size distribution of this nanovesicles were confirmed by NTA (Nanosight Traking Analysis).
**b)** In the case of biotinylated-tagged recombinant proteins nanovesicles composition was
   - Span 60 (Sorbitane monostea- rate),
   - Tween 60 (Polyoxyethylenesorbitan monolaurate)
   - Cholesterol and DSPE-2000-MAL (1,2-distearoyl-sn- glycero-3-phosphoethanolamine-N-[maleimide(polyethy- lene glycol)-2000] (ammonium salt))
      with the following molar ratios: 0.5:0.5:1:0.01.
   - 1,2 dioleoyl-sn-glycerol phosphoethanolamine modified with carboxyfluorescein molecules was included in the formulation to generate fluorescent nanovesicles.

The components were hydrated with 2 mL PBS 20 mM pH 7 at 60°C. The mixture was then shaken for two minutes and sonicated using a bath sonicator for 15 min to produce nanovesicles with a size range between 50 and 300 nm.

Surface modification of nanovesicles was performed by bioconjugation with streptavidin (SA) or avidin through thiol activated groups within the molecules of the bilayer, thereby generating a covalent bond between (strept)avidin molecules and the nanovesicles. One milligram of a sulfhydryl-containing protein (SA or avidin) was disolved in a 4 mL nanovesicles solution and incubated for 2 h after previous deprotection of the reactive groups of the SATA activated strept(avidin) with hydroxylamine-HCL. To remove residual hydroxylamine and deacylation products, samples were desalted by gel filtration using Sephadex G-75. The bioconjugated nanovesicles were purified using a column of Agarose 6%. The amount of unbound (strep)avidin was measured by a Bradford assay in the filtrated fraction. Final nanovesicle solutions were analysed using Dynamic Light Scattering (DLS) and NTA to determine their size and concentration, respectively. The change in the Z potential of the bioconjugated nanovesicles confirmed the presence of (strep)avidin on the surface of those. In the nanovesicles stocks used in the immunization the amount of (strept)avidin conjugated to the surface was reported to be 232 µg/ml of avidin in the fluorescent nanovesicles and 224 µg/ml of SA in non-fluorescent nanovesicles.

### Decoration of lipid nanovesicles with recombinant proteins.

For the binding of recombinant proteins, polyhis tagged recombinant KMP-11, CD9mLEL or Spike were used. The anchoring of polyhistidine tag to Ni(II) occurs on the imidazole nitrogen close to the N-terminal and provide an stable coupling site. For this coupling, nanovesicles and proteins (in an excess of 2x of the possible protein binding sites that were expected to be ½ of the total NTA lipids i.e. 15 µM binding sites) were incubated overnight, at 4°C, in an orbital rotator.

In the case of Avi-tagged CD9mLEL and biotinylated KMP-11 we took advantage of the high-affinity interaction between (strept)avidin-biotin molecules to decorate the surface of the nanovesicles with the recombinant large extracellular loop of CD9 tetraspanin (LEL) and KMP-11. Recombinant biotinylated molecules were also added at a double of concentration of the expected binding sites, as determined by determination of the amount of unbound (strep)avidin measured by a Bradford assay.

Unbounded recombinant molecules were removed by SEC (size-exclusion chromatography) using 4BCL agarose (Agarose Bead Technologies).

The concentration of recombinant proteins bound to the surface of the final nanovesicles was estimated by western blot analysis. Coupling of proteins to nanovesicles was confirmed by flow cytometry.

### Animals

Five-week-old female wild type BALB/cOlaHdr mice were obtained from ENVIGO CRS SA. All animals were maintained under specific pathogen-free conditions, in ventilated cages as well as with food and water *adlibitum.*

All procedures carried out with experimental animals are in accordance with European Directive 2010/63/UE and were authorized by the Government of the Comunidad de Madrid (PROEX 121/14 and PROEX 134/19) and revised by the Animal Care and Use Committee at the Centro de Biologia Molecular Severo Ochoa (CBMSO) as well as By the Bioethical Committee of the Spanish Consejo Superior de Investigaciones Científicas (CSIC) under reference 795/2019.

### Immunization protocols

Solutions of soluble recombinant immunogenic proteins (sKMP-11 and sSpike), or nanovesicles decorated only with recombinant CD9LEL protein (nCD9), nanovesicles decorated only with antigenic protein (nKMP-11 and nSpike) and nanovesicles decorated with both antigenic protein and CD9 protein (nCD9+ KMP-11 and nCD9 + Spike) were prepared in PBS. 400 µl of the different preparations containing in all cases 10 µg of antigenic protein, were intraperitoneally injected in mice using a 22-G needle.

In the experiment with biotin-tag, only one immunization was performed and samples were collected from the submandibular vein 1 day before first immunization (pre-immune samples) and 3 weeks after first immunization, via cardiac puncture, after mice euthanasia.

In the experiment with His-tagged proteins, mice were immunized twice at 2 week intervals and bleeds were collected from the submandibular vein 1 day before first immunization and 7 weeks after first immunization, by cardiac puncture, after mice euthanasia. Blood samples were processed to obtain serum samples that were stored in glycerol (1:1) at 20°C.

### Enzyme-linked immunosorbentent assays (ELISA) for detection of antigen-specific serum-IgGs

ELISAs were established for detection of serum total IgG and IgG isotypes (IgG1, IgG2a, IgG2b and IgG3) specific for KMP-11, Spike and CD9.

200 ng/well of the different recombinant proteins (KMP-11, Spike and CD9) were used to coat over night at 4 °C Corning Costar^{®} 96 assay plates. Plates were washed four times with 200 µl/well of washing solution (WS: PBS-0,05% Tween20) and then blocked with blocking solution (BS: PBS-0,05% Tween20-5% milk) for one hour. 200 µl of serial dilutions (1:500, 1:1,000. 1:2,000, 1:4,000, 1;8,000, 1:16,000, 1:32,000, 1:64:000) in blocking medium of each serum were added to the ELISA plates and incubated for 2h at RT. Plates were then washed four times with WS and incubated for 1 h at RT with a 1:2,000 dilution of a HRP-conjugated goat anti-mouse anti-total IgG, -IgG1 or -IgG2a antibody (Nordic MUbio) or a 1:4,000 dilution of a HRP-conjugated goat anti-mouse IgG2b or IgG3 antibody (Abcam). The plates were washed four times with WS and 100 µl of Thermo Scientific OPD (o-phenylenediamine dihydrochloride) solution substrate for horseradish peroxidase (HRP) were added. The reaction was stopped with 50 µl of 2,5 M H2SO4 after 10 min. Absorbance was measured at 490 nm in a microplate reader Tecan GENios. Pooled sera from not immunized mice, wells without serum and without HRP-conjugated goat anti-mouse IgG antibody were used as negative controls.

## Claims

1. A pharmaceutical composition, preferably a vaccine composition, comprising a lipid nanoparticle, preferably a nanovesicle, and optionally an excipient, wherein the lipid nanoparticle, preferably a nanovesicle, comprises the large extracellular loop (LEL) of CD9 of SEQ ID NO 2 displayed on its surface or an amino acid sequence having at least 75%, 80%, 85%, 90%, 91%, 92%, 93%, 94%, 95%, 96%, 97%, 98%, 99% or 100% identity to SEQ ID NO 2 displayed on its surface.

2. The pharmaceutical composition of claim 1, wherein the lipid nanoparticle, preferably the nanovesicle, of the pharmaceutical composition further comprises polypeptide immunogens displayed on its surface different from the large extracellular loop (LEL) of CD9 of SEQ ID NO 2 and different from an amino acid sequence having at least 75%, 80%, 85%, 90%, 91%, 92%, 93%, 94%, 95%, 96%, 97%, 98%, 99% or 100% identity to SEQ ID NO 2.

3. The pharmaceutical composition according to anyone of claims 1 to 2, wherein the lipid nanoparticle is a nanovesicle.

4. The pharmaceutical composition according to anyone of claims 1 to 3, wherein the large extracellular loop (LEL) of CD9 and the polypeptides immunogens are linked or bound to a lipid component of the lipid nanoparticle.

5. The pharmaceutical composition according to the preceding claim, wherein the lipid component is one or more of phosphatidylcholine, cholesterol, phosphatidylglycerol, phosphatidyl- ethanolamine, phosphatidylserine, phosphatidic acid, sphingomyelin or derivatives of these lipids.

6. The pharmaceutical composition according to claim 5, wherein the lipid components are phosphatidylcholine and cholesterol.

7. The pharmaceutical composition according any one of claims 2 to 6, wherein the polypeptide immunogens can be selected from any proteins or polypeptides from pathogens against which an elicited immune response is desired.

8. The pharmaceutical composition according any one of claims 2 to 6, wherein the polypeptide immunogens are derived from any viruses, bacteria or other pathogenic organisms such as unicellular or multicellular parasites.

9. The pharmaceutical composition according any one of claims 2 to 6, wherein the polypeptide immunogens are selected from an endogenous protein from a mammalian host (e.g., human), against which an elicited immune response is desired.

10. The pharmaceutical composition according any one of claims 2 to 6, wherein the polypeptide immunogens are selected from recombinant KMP-11 from *Leishmania* parasite or the recombinant Spike, in the case of SARs-Cov-2 virus.

11. The pharmaceutical composition according to any of claims 1 to 10, for use as a medicament.

12. The pharmaceutical composition according to any of claims 1 to 10, for use as an immunogenic composition capable of inducing a measurable CTL response against the immunogenic polypeptide displayed on the surface of the lipid nanoparticle, and/or inducing a measurable B cell response (such as production of antibodies) against the immunogenic polypeptide.

13. The pharmaceutical composition according to any of claims 1 to 10, wherein the large extracellular loop (LEL) of CD9 of SEQ ID NO 2 or the amino acid sequence at least 75%, 80%, 85%, 90%, 91%, 92%, 93%, 94%, 95%, 96%, 97%, 98%, 99% or 100% identical to SEQ ID NO 2, associated to or displayed on its surface of the lipid nanoparticle, is for use as an immunologic adjuvant of one or more polypeptide immunogens also displayed on the surface of the lipid nanoparticles.

14. The pharmaceutical composition according to claim 13, wherein the large extracellular loop (LEL) of CD9 of SEQ ID NO 2 or the amino acid sequence at least 75%, 80%, 85%, 90%, 91%, 92%, 93%, 94%, 95%, 96%, 97%, 98%, 99% or 100% identical to SEQ ID NO 2, associated to or displayed on its surface of the lipid nanoparticle, is for use as an immunologic adjuvant of the polypeptide immunogens displayed on the surface of the lipid nanoparticles.
